# EUROPEAN PATENT APPLICATION

(11) **EP 4 553 479 A1**
(43) Date of publication of application: **14.05.2025**
(21) Application number: 22950207.5
(22) Date of filing: 05.07.2022
(51) Int. Cl.: G01N 1/10, G01N 33/48

(54) **SPECIMEN COLLECTION DEVICE AND SPECIMEN COLLECTION KIT**

(71) Applicant: Blue Industries Inc., Tokyo 130-0013 (JP)
(72) Inventor: KUJI, Tomoaki, Tokyo 130-0013 (JP)
(74) Representative: Reddie & Grose LLP
(86) International application number: PCT/JP2022/026771
(87) International publication number: WO 2024/009413

(57) **Abstract**

[Problem] Provided are a specimen collection device and a specimen collection kit capable of regulating outflow of a specimen into a collection container.

[Solution] A specimen collection device according to an embodiment includes: a conduit configured to cause a specimen flowing into a first end portion from an outside of a container having an inside depressurized for collecting the specimen to flow out into the container from a second end portion disposed so as to be opposite to the first end portion; a holding member configured to hold the container and the conduit; and a regulating member configured to cause the specimen to flow out into the container when being opened and regulate outflow of the specimen into the container when being closed.

## Description

### Technical Field

The present disclosure relates to a specimen collection device and a specimen collection kit that collect a specimen of an animal including a human for a specimen test.

### Background Art

For clinical tests in medical care for humans and other animals, a specimen test for testing a liquid specimen such as blood or urine may be performed. For example, when the specimen is blood, a method may be adopted in which blood is collected from a subject with a syringe, and a syringe needle of the syringe is inserted into a lid of a collection container such as a depressurized test tube to cause the blood to flow into the collection container. For example, Patent Literatures 1 and 2 disclose specimen collection devices that collect blood as a specimen.

However, when a specimen collection device as disclosed in these literatures is used, it is not easy to perform an operation for regulation to start or stop outflow of a specimen into the collection container.

### Citation List

### Patent Literature

Patent Literature 1: WO 2014/185902 A
Patent Literature 2: JP 2008-302077 A

### Summary of Invention

### Technical Problem

Therefore, based on the above-described techniques, the present disclosure provides a specimen collection device and a specimen collection kit capable of regulating outflow of a specimen into a collection container according to various embodiments.

### Solution to Problem

In order to solve the above problem, one aspect of the present disclosure is
(1) a specimen collection device including: a conduit configured to cause a specimen flowing into a first end portion from an outside of a container having an inside depressurized for collecting the specimen to flow out into the container from a second end portion disposed so as to be opposite to the first end portion; a holding member configured to hold the container and the conduit; and a regulating member configured to cause the specimen to flow out into the container when being opened and regulate outflow of the specimen into the container when being closed.

A preferred aspect of the present disclosure is
(2) the specimen collection device according to (1), in which the specimen is a body fluid of an animal including a human.

A preferred aspect of the present disclosure is
(3) the specimen collection device according to (1), in which the conduit includes a hollow pipe and a hollow needle tube to be inserted into the container from a lid of the container.

A preferred aspect of the present disclosure is
(4) the specimen collection device according to (3), in which the regulating member regulates, in the pipe, outflow of the specimen into the container.

A preferred aspect of the present disclosure is
(5) the specimen collection device according to (3), in which the regulating member regulates outflow of the specimen into the container by closing the pipe.

A preferred aspect of the present disclosure is
(6) the specimen collection device according to (3), in which the pipe is configured to be crushed at least in a short direction of a cross section of the pipe by pressurization, and the regulating member regulates outflow of the specimen into the container by pressurizing the pipe in the short direction.

A preferred aspect of the present disclosure is
(7) the specimen collection device according to (1), in which the regulating member is disposed in the holding member.

A preferred aspect of the present disclosure is
(8) the specimen collection device according to (1), in which the first end portion is connected to a tube having a distal end drawn into another container different from the container, and the regulating member regulates outflow of the specimen into the container in the tube.

A preferred aspect of the present disclosure is
(9) the specimen collection device according to (8), in which the regulating member is disposed away from the holding member.

Another aspect of the present disclosure is
(10) a specimen collection kit including: a collection container having an inside depressurized for collecting a specimen and sealed with a lid; and the specimen collection device according to (1).

### Advantageous Effects of Invention

According to various embodiments of the present disclosure, it is possible to provide a specimen collection device and a specimen collection kit capable of regulating outflow of a specimen into a collection container.

Note that the above effect is merely exemplary for convenience of description, and is not restrictive. In addition to or instead of the above effect, any effect described in the present disclosure and an effect obvious to those skilled in the art can also be exhibited.

### Brief Description of Drawings

Fig. 1 is a diagram exemplifying a configuration of a specimen collection device 12 according to a first embodiment and a specimen collection kit 1 including the specimen collection device 12.
Fig. 2A is a first diagram exemplifying the configuration of the specimen collection device 12 according to the first embodiment and the specimen collection kit 1 including the specimen collection device 12.
Fig. 2B is a second diagram exemplifying the configuration of the specimen collection device 12 according to the first embodiment and the specimen collection kit 1 including the specimen collection device 12.
Fig. 3A is a first diagram exemplifying the configuration of the specimen collection device 12 according to the first embodiment and the specimen collection kit 1 including the specimen collection device 12.
Fig. 3B is a second diagram exemplifying the configuration of the specimen collection device 12 according to the first embodiment and the specimen collection kit 1 including the specimen collection device 12.
Fig. 3C is a third diagram exemplifying the configuration of the specimen collection device 12 according to the first embodiment and the specimen collection kit 1 including the specimen collection device 12.
Fig. 4 is a diagram exemplifying a method for using the specimen collection kit 1 illustrated in Figs. 3A and 3B.
Fig. 5A is a diagram illustrating a configuration of a specimen collection device 13 that can substitute for the specimen collection device 12 in the specimen collection kit 1 according to the first embodiment.
Fig. 5B is a diagram illustrating the configuration of the specimen collection device 13 that can substitute for the specimen collection device 12 in the specimen collection kit 1 according to the first embodiment.
Fig. 5C is a diagram illustrating an example of an abutting member that can be used in the specimen collection kit 1 according to the first embodiment.
Fig. 6A is a first diagram illustrating a configuration of a specimen collection device 14 that can substitute for the specimen collection device 12 in the specimen collection kit 1 according to the first embodiment.
Fig. 6B is a second diagram illustrating the configuration of the specimen collection device 14 that can substitute for the specimen collection device 12 in the specimen collection kit 1 according to the first embodiment.
Fig. 7A is a first diagram illustrating a configuration of a specimen collection device 15 that can substitute for the specimen collection device 12 in the specimen collection kit 1 according to the first embodiment.
Fig. 7B is a second diagram illustrating the configuration of the specimen collection device 15 that can substitute for the specimen collection device 12 in the specimen collection kit 1 according to the first embodiment.
Fig. 8 is a diagram exemplifying a configuration of a specimen collection device 22 according to a second embodiment and a specimen collection kit 20 including the specimen collection device 22.
Fig. 9 is a diagram exemplifying a configuration of a specimen collection kit 26 including the specimen collection device 22 as a modification of the second embodiment.
Fig. 10A is a first diagram exemplifying a configuration of a specimen cylinder 3 according to a third embodiment that can be used for specimen collection by the specimen collection devices 12, 14, 15, and 22 illustrated in the first and second embodiments.
Fig. 10B is a second diagram exemplifying the configuration of the specimen cylinder 3 according to the third embodiment that can be used for specimen collection by the specimen collection devices 12, 14, 15, and 22 illustrated in the first and second embodiments.
Fig. 11A is a first diagram exemplifying the configuration of the specimen cylinder 3 according to the third embodiment used for specimen collection by the specimen collection kit 1.
Fig. 11B is a second diagram exemplifying the configuration of the specimen cylinder 3 according to the third embodiment used for specimen collection by the specimen collection kit 1.
Fig. 12 is a diagram illustrating a method for collecting a specimen, put in the specimen cylinder 3 illustrated in Figs. 11A and 11B, using the specimen collection kit 1.
Fig. 13A is a first diagram exemplifying a cross section of a part of a specimen collection device 40 according to a fourth embodiment and a specimen collection kit 4 including the specimen collection device 40 in an enlarged manner.
Fig. 13B is a second diagram exemplifying a cross section of a part of the specimen collection device 40 according to the fourth embodiment and the specimen collection kit 4 including the specimen collection device 40 in an enlarged manner.
Fig. 13C is a third diagram exemplifying a cross section of the specimen collection device 40 according to the fourth embodiment and a part of the specimen collection kit 4 including the specimen collection device 40 in an enlarged manner.
Fig. 14 is a diagram exemplifying a cross section of a specimen cup 8 in which a specimen collection kit 1 according to a fifth embodiment is used.
Fig. 15 is a diagram illustrating a modification of the fifth embodiment.

### Description of Embodiments

Hereinafter, various embodiments for carrying out a specimen collection device and a specimen collection kit of the present disclosure will be described in detail. Note that the following embodiments are examples for describing the present disclosure, and the present disclosure is not limited only to the embodiments.

### First Embodiment

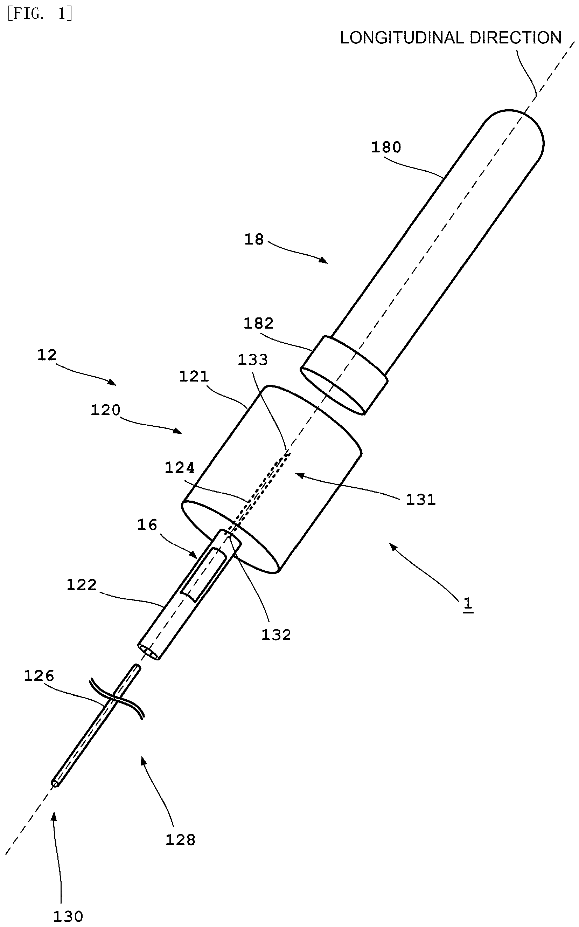

First, a first embodiment will be described. Fig. 1 is a diagram exemplifying a configuration of a specimen collection device 12 according to a first embodiment and a specimen collection kit 1 including the specimen collection device 12. Specifically, Fig. 1 is a perspective view illustrating components of the specimen collection kit 1. According to Fig. 1, the specimen collection kit 1 includes the specimen collection device 12 and a collection container 18.

The specimen collection kit 1 is used to collect a specimen in a predetermined container. Here, in the present disclosure, the specimen includes a sample containing a body fluid such as blood, urine, tears, sweat, nasal mucus, sputum, saliva, bile, digestive fluid, lymph, or spinal fluid collected from a living body, an organ, a tissue, or a cell (including a cultured cell) of a living body, a nucleic acid, a protein, or a virus, a sample collected from outside of a living body, such as groundwater, river, ocean, rainwater, beverage, food, or medicine, or a combination thereof, preferably includes a sample containing a body fluid such as blood, urine, tears, sweat, nasal mucus, sputum, saliva, bile, digestive fluid, lymph, or spinal fluid collected from a living body, an organ, a tissue, or a cell (including a cultured cell) of a living body, a nucleic acid, a protein, or a virus, or a combination thereof, and more preferably includes a body fluid such as blood, urine, tears, sweat, nasal mucus, sputum, saliva, bile, digestive fluid, lymph, or spinal fluid collected from a living body. Note that the specimen is typically a liquid derived from a living body, but may be, for example, a gas such as air. The living body includes invertebrates, plants, bacteria, viruses, and the like, in addition to spinal animals such as mammals including humans, birds, reptiles, and amphibians.

Such a specimen collection kit 1 is used at various places such as a medical site, a laboratory, a test site, an environmental survey, a farm, a livestock farm, and a field work site, preferably at a medical site, a laboratory, or a test site, and more preferably at a medical site. Typically, the specimen collection kit 1 is used to cause blood collected by a syringe from a living body at a medical site to flow into a container included in the specimen collection kit 1 via the specimen collection device 12 and to collect the blood in the container. Note that this use example is an example, and a method of use thereof is appropriately changed according to the type of specimen and a site where the specimen is used.

The specimen collection device 12 includes a conduit 128, a holding member 120, and a regulating member 16. The holding member 120 includes a main body 121 and a pipe holding member 122. The conduit 128 includes a first end portion 130 that allows a specimen to flow into the conduit 128 and a second end portion 131 disposed so as to be opposite to the first end portion 130. The second end portion 131 is disposed so as to be located inside the collection container 18, and is constructed so that a specimen that has flowed through the conduit 128 flows out into the collection container 18 from the second end portion 131. Therefore, the conduit 128 is formed into a hollow shape such that a specimen can flow therethrough. As an example, the conduit 128 is formed of a metal material such as stainless steel, aluminum, an aluminum alloy, titanium, or a titanium alloy, a synthetic resin material such as a thermoplastic resin such as a polyolefin including polyethylene (PE), polypropylene (PP), polybutene, an ethylene-propylene copolymer, an ethylene-vinyl acetate copolymer, and a mixture thereof, an acrylonitrile-styrene resin (AS), an acrylonitrile-butadiene-styrene resin (ABS), polyvinyl chloride (PVC), a methacrylic resin (PMMA), polyethylene terephthalate (PET), polyamide (PA), a polyamide elastomer, polyester, a polyester elastomer, polyurethane (PU), or a fluororesin, or a material formed of a silicone rubber, a latex rubber, a vegetable plastic, waterproof paper, or a combination thereof.

In addition, in order to perform regulation by the regulating member 16 more effectively, at least a part of the conduit 128 desirably includes a flexible region formed of a synthetic resin material such as a thermoplastic resin such as a polyolefin including polyethylene (PE), polypropylene (PP), polybutene, an ethylene-propylene copolymer, an ethylene-vinyl acetate copolymer, and a mixture thereof, polyvinyl chloride (PVC), polyamide (PA), a polyamide elastomer, polyester, a polyester elastomer, polyurethane (PU), or a fluororesin, a silicone rubber, a latex rubber, or the like.

The conduit 128 may be formed of a single material selected from the materials listed above, but can be formed of a combination of a plurality of materials as illustrated in Fig. 1. According to Fig 1, the conduit 128 includes a needle tube 124 and a pipe 126. The needle tube 124 is formed of a synthetic resin material such as a thermoplastic resin such as polyethylene (PE), polypropylene (PP), polystyrene (PS), an acrylonitrile-styrene resin (AS), an acrylonitrile-butadiene-styrene resin (ABS), polyvinyl chloride (PVC), a methacrylic resin (PMMA), or polyethylene terephthalate (PET), a metal material such as stainless steel, aluminum, an aluminum alloy, titanium, or a titanium alloy, waterproof paper, a vegetable plastic, or a material formed of a combination thereof. The needle tube 124 is formed into a hollow cylindrical shape such that a specimen can flow therethrough. It is formed into a thickness suitable for specimen collection. The needle tube 124 is disposed such that one end 132 thereof is housed inside the main body 121. The other end 133 located in a direction opposite to the one end 132 of the needle tube 124 is formed into a needle shape oblique to a longitudinal direction indicated by a dotted line in Fig. 1 so as to be easily inserted into the collection container 18. That is, the other end 133 side of the needle tube 124 is inserted into the collection container 18, and a specimen flows out toward the collection container 18 from the other end 133. Note that, in the present embodiment, the case where the needle tube 124 is used in order to insert the conduit 128 into the collection container 18 has been described, but it is needless to say that the conduit 128 may be inserted with a cylindrical object other than the needle tube 124.

As an example, the pipe 126 is formed of a synthetic resin material such as a thermoplastic resin such as a polyolefin including polyethylene, polypropylene, polybutene, an ethylene-propylene copolymer, an ethylene-vinyl acetate copolymer, and a mixture thereof, a polyvinyl chloride resin, polyamide, a polyamide elastomer, polyester, a polyester elastomer, polyurethane, or a fluororesin, or a flexible material such as a silicone rubber or a latex rubber. The pipe 126 is formed to be hollow and have a thickness and a length suitable for specimen collection such that a specimen can flow therethrough. Note that the length of the pipe 126 can be set to a length suitable for specimen collection by a user who intends to collect a specimen by cutting the pipe 126 at a medical site. The pipe 126 preferably has softness and flexibility in a short direction of a cross section of the pipe 126 to such an extent that the pipe 126 is deformed and crushed to close the hollow portion when being pressurized in the short direction by the regulating member 16. In addition, the pipe 126 has a restoration property to such an extent that the pipe 126 returns to the original shape and opens the hollow portion when pressurization is released after the pipe 126 is pressurized in the short direction by the regulating member 16 and crushed to close the hollow portion.

The collection container 18 includes a container main body 180, such as a test tube, and a lid 182 thereof. As an example, the container main body 180 is formed of a thermoplastic resin such as polyethylene, polypropylene, polystyrene, polyethylene terephthalate, polymethyl methacrylate, or polyacrylonitrile, a thermosetting resin such as an unsaturated polyester resin, an epoxy resin, or an epoxy-acrylate resin, a modified natural resin such as cellulose acetate, cellulose propionate, ethyl cellulose, or ethyl chitin, a glass material such as a silicate glass (such as soda lime glass, phosphosilicate glass, or borosilicate glass) or quartz glass, or a combination thereof. Desirably, the container main body 180 has a volume suitable for the amount of a specimen to be collected, and the inside thereof is depressurized with respect to the outside air for specimen collection. An air pressure inside the container main body 180 may be appropriately determined according to the type, amount, and the like of a specimen to be collected.

The lid 182 is formed of a synthetic resin, an elastomer, a rubber, a metal foil, or the like. Examples of such a rubber include a butyl rubber and a halogenated butyl rubber. Examples of such a metal foil include an aluminum foil. The lid 182 is preferably formed of a butyl rubber from a viewpoint of ensuring airtightness of the container main body 180. The lid 182 seals an opening of the container main body 180 and holds depressurization of the container main body 180. The other end 133 of the needle tube 124 is inserted into the lid 182 by a user using the specimen collection kit 1, and a distal end thereof is located in the container main body 180, whereby a specimen that has flowed into the needle tube 124 from the pipe 126 flows out into the container main body 180.

The holding member 120 includes the main body 121 and the pipe holding member 122. As an example, the holding member 120 is formed of a thermoplastic resin such as polyethylene, polypropylene, polystyrene, polyethylene terephthalate, polymethyl methacrylate, or polyacrylonitrile, a thermosetting resin such as an unsaturated polyester resin, an epoxy resin, or an epoxy-acrylate resin, a modified natural resin such as cellulose acetate, cellulose propionate, ethyl cellulose, or ethyl chitin, a glass material such as a silicate glass (such as soda lime glass, phosphosilicate glass, or borosilicate glass) or quartz glass, or a combination thereof. Note that the main body 121 and the pipe holding member 122 may be integrally formed of any of the above exemplified materials, or may be formed by joining after performing molding by combining a plurality of materials.

The main body 121 has an opening at one end in a longitudinal direction thereof so that at least a part of the collection container 18 is inserted thereinto via the opening and the collection container 18 can be housed therein. An outer periphery of the main body 121 in a short direction thereof is formed into a shape along an outer periphery of the collection container 18 in a short direction thereof. That is, the main body 121 typically has a substantially cylindrical shape extending in a direction in which the collection container 18 is inserted. The inside of the main body 121 is formed so as to have a predetermined depth along a longitudinal direction of the collection container 18.

The main body 121 has a connection hole having a diameter slightly larger than a diameter of the conduit 128 at substantially a center on the other end side located in a direction opposite to the one end having the opening. The main body 121 is connected to one end of the pipe holding member 122 in the connection hole.

The pipe holding member 122 is formed into a hollow substantially cylindrical shape having a diameter slightly larger than the diameter of the conduit 128. One end of the pipe holding member 122 is connected to the connection hole of the main body 121. The other end of the pipe holding member 122 located in a direction opposite to the one end is opened so that the conduit 128 can be inserted therein via the opening. That is, the conduit 128 is inserted into the opening formed at the other end of the pipe holding member 122, passes through the inside of the hollow pipe holding member 122, and extracted from the connection hole. As a result, as illustrated in Fig. 1, while the pipe 126 of the conduit 128 is held by the pipe holding member 122, the needle tube 124 is exposed to the inside of the main body 121.

In the present embodiment, the regulating member 16 is formed in at least a part of the pipe holding member 122, and regulates passage of a specimen through the pipe 126 by pressurization on the pipe 126 passing through the pipe holding member 122. Note that a detailed configuration thereof will be described later, but the regulation by pressurization is an example, and it is needless to say that regulation may be performed by other methods.

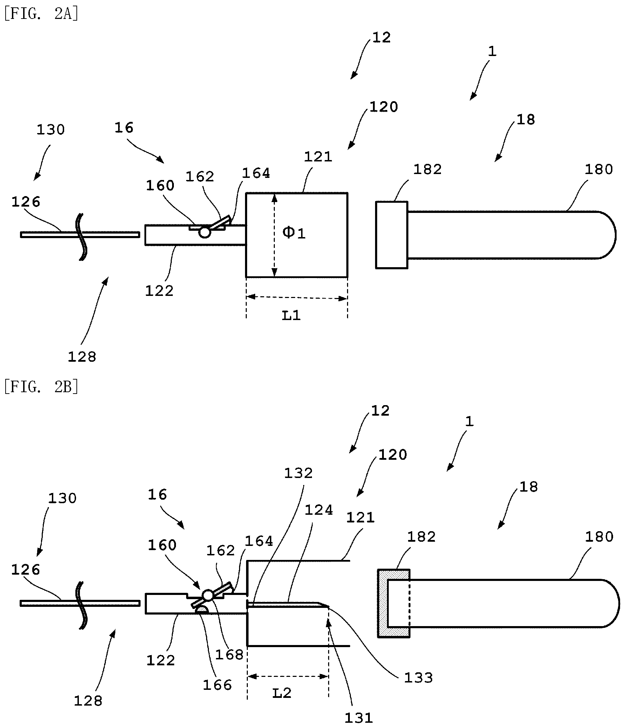

Figs. 2A and 2B are diagrams exemplifying a configuration of the specimen collection device 12 according to the first embodiment and the specimen collection kit 1 including the specimen collection device 12. Specifically, Fig. 2A is a diagram illustrating a structure of a side surface of the specimen collection kit 1 including the regulating member 16, and Fig. 2B is a diagram illustrating a cross section of the specimen collection kit 1 including the regulating member 16 in a longitudinal direction thereof. Note that Φ1 illustrated in Figs. 2A and 2B represents the inner diameter of the main body 121 illustrated in Fig. 1, and L1 represents the length of the main body 121 in a longitudinal direction thereof, that is, the depth of the main body 121. L2 represents the length of the needle tube 124 in a longitudinal direction thereof. Since the one end 132 of the needle tube 124 is housed inside the main body 121, L1 and L2 have a relationship of L1 > L2. As illustrated in Figs. 2A and 2B, the regulating member 16 is disposed at an opening 160 formed in the pipe holding member 122, and includes, as an example, a lever member 162, an elastic member 164, a protruding member 166, and support members 168. The support members 168 are formed of, for example, the same material as the pipe holding member 122, and two support members 168 are attached to edge portions of the opening 160 so as to face each other in a short direction of the pipe holding member 122. The support members 168 support respective sides (long sides) of the lever member 162 in a short direction thereof to be rotatable about the support members 168.

The elastic member 164 is, for example, a member to which elasticity is imparted by bending a stainless plate or rod. As an example of such an elastic member 164, a leaf spring is used. The elastic member 164 is disposed between the pipe holding member 122 and one end of the lever member 162, and biases the one end of the lever member 162 upward with respect to the pipe holding member 122. As a result, the lever member 162 rotates about the support members 168, and the other end of the lever member 162 located in a direction opposite to the one end is biased downward with respect to the pipe holding member 122. In addition, the other end of the lever member 162 is biased downward with respect to the pipe holding member 122 by the elastic member 164 to abut on the protruding member 166 disposed inside the pipe holding member 122 (when the conduit 128 is not inserted into the pipe holding member 122). That is, when a force F sufficient to separate the other end of the lever member 162 from the protruding member 166 is not applied to the one end of the lever member 162 by a user, the elastic member 164 applies a force in a direction in which the other end of the lever member 162 rotates in a direction of the protruding member 166. As a result, in a state where the pipe 126 is not inserted between the lever member 162 and the protruding member 166, at least a part of the other end of the lever member 162 inside the pipe holding member 122 abuts on the protruding member 166. Furthermore, when the pipe 126 of the conduit 128 is inserted into the pipe holding member 122, the other end of the lever member 162 is biased in the direction of the protruding member 166 by the elastic member 164, and thus the pipe 126 is deformed so as to be crushed in a short direction thereof by the other end and the protruding member 166.

On the other hand, in this state, when the force F is applied by a user, the other end of the lever member 162 is rotated in a direction away from the protruding member 166 by the force F against an elastic force of the elastic member 164 so that the other end of the lever member 162 and the protruding member 166 are separated from each other. As a result, the pipe 126, deformed so as to be crushed in the short direction by the other end of the lever member 162 and the protruding member 166 by being biased by the elastic member 164, is released from the pressurization and returns to the original shape of the pipe 126 before being deformed. Note that the elastic member 164 does not need to be formed of stainless steel, and it is also possible to use a material selected from the group consisting of an alloy containing iron other than stainless steel, an alloy containing copper, titanium, and Inconel (registered trademark). In addition, although the leaf spring has been described as an example, the elastic member 164 only needs to be able to bias the lever member 162 in a desired direction, and may be any of a coil spring, a spiral spring, a rubber, and the like. In addition, the pipe 126 only needs to be able to be deformed, and the protruding member 166 does not necessarily have to be disposed.

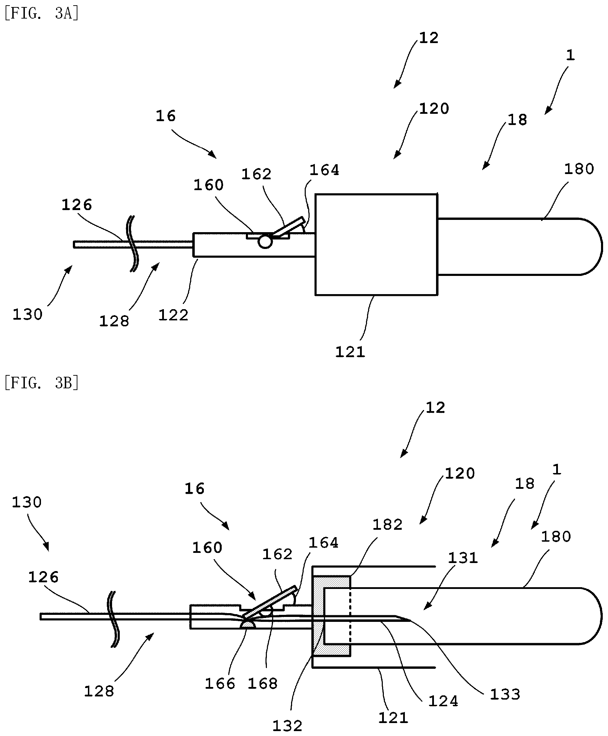

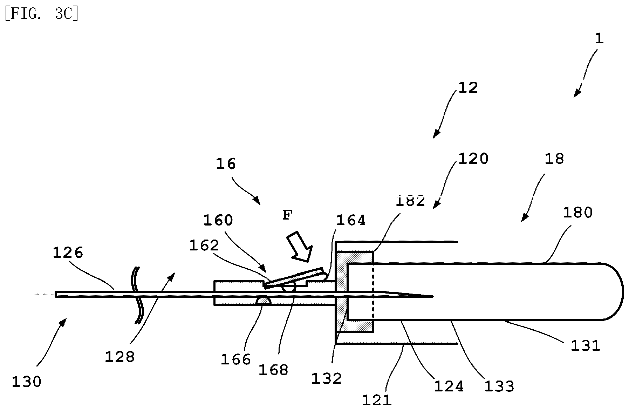

Figs. 3A to 3C are diagrams exemplifying the configuration of the specimen collection device 12 according to the first embodiment and the specimen collection kit 1 including the specimen collection device 12. Specifically, Fig. 3A is a diagram illustrating a structure of a side surface in a state where the collection container 18 and the conduit 128 are connected to each other and held by the holding member 120. Figs. 3B and 3C are diagrams illustrating a structure in a cross section in a state where the collection container 18 and the conduit 128 are connected to each other and held by the holding member 120.

Here, as illustrated in Figs. 3A and 3B, the main body 121 of the holding member 120 has a cup-like shape having an opening in a direction in which the collection container 18 is inserted, a bottom surface located in a direction opposite thereto, and a wall surface extending from the bottom surface in a direction of the opening. The conduit 128 is inserted from an opening formed at the other end of the pipe holding member 122 until at least a distal end of the needle tube 124 disposed at one end (second end portion) of the conduit 128 reaches a position exposed to the inside from a bottom surface of the cup-like-shaped main body 121. At this time, since the lever member 162 is biased in the direction of the protruding member 166 by the elastic member 164 and abuts on the protruding member 166, it is necessary to apply a force F in a direction opposite thereto by a user to release the abutment on the protruding member 166. As a result, the conduit 128 is held with respect to the holding member 120.

As described above, the main body 121 of the holding member 120 has an opening in a direction in which the collection container 18 is inserted. The collection container 18 is inserted into the holding member 120 from the opening until the lid 182 of the collection container 18 comes into contact with or close to the bottom surface of the main body 121 located in a direction opposite to the opening. At this time, as the collection container 18 is inserted into the main body 121, the lid 182 of the collection container 18 comes into contact with a distal end of the needle tube 124 of the conduit 128 exposed to the inside of the main body 121, and the collection container 18 is further inserted (a force is applied), whereby the distal end of the needle tube 124 is inserted into and penetrates the lid 182. When the collection container 18 is inserted until the lid 182 of the collection container 18 comes into contact with or close to the bottom surface of the main body 121, the second end portion 131 of the conduit 128 is located inside the container main body 180 of the collection container 18. As a result, the conduit 128 and the collection container 18 are connected to each other while maintaining a depressurized state of the collection container 18, and the conduit 128 and the collection container 18 are held by the holding member 120. Note that it is described that the holding member 120 holds the conduit 128 and the collection container 18. However, this does not mean only a case where the holding member 120 holds the conduit 128 and the collection container 18 fixedly, but also includes a case where each member is located in any space of the pipe holding member 122, such as a case where the conduit 128 is simply inserted into the pipe holding member 122 or a case where the collection container 18 is simply housed in the main body 121. In addition, the conduit 128 only needs to be able to form a structure in which a specimen is inserted from the first end portion, passes through the inside of the conduit 128, and can be discharged to the outside from the second end portion. That is, the conduit 128 may be formed by fixing the needle tube 124 to the main body 121 of the holding member 120 in advance and then connecting the pipe 126 to an end of the needle tube 124 on a side opposite to an end inserted into the collection container 18.

Here, Figs. 3A and 3B each illustrate a state in which the conduit 128 and the collection container 18 are connected to each other as described above and held by the holding member 120. In this state, the other end of the lever member 162 of the regulating member 16 is biased by the elastic member 164 in a direction of abutting on the protruding member 166. Therefore, at least a part (specifically, the pipe 126) having flexibility of the conduit 128 is deformed so as to be crushed in a cross section in a short direction thereof. Therefore, in this state, the conduit 128 regulates passage of the specimen inserted from one end (first end portion) thereof through the inside of the conduit 128 and outflow of the specimen from the other end (second end portion) thereof.

Next, Fig. 3C illustrates a case where the regulation by the regulating member is released by applying a force F to one end of the lever member 162 in a state where the conduit 128 and the collection container 18 are connected to each other as described above and held by the holding member 120. Specifically, a user applies a force F to one end of the lever member 162, whereby the lever member 162 is rotated about the support members 168, and the other end of the lever member 162 moves in a direction opposite to the direction of the protruding member 166. As a result, the cross section of the pipe 126 deformed so as to be crushed by the other end and the protruding member 166 is released from the pressurization and returns to a normal cross-sectional shape. Therefore, in this state, the regulation of the passage of the specimen by the regulating member 16 is released, and the conduit 128 allows the specimen inserted from one end (first end portion) thereof to pass through the inside of the conduit 128 and to flow out from the other end (second end portion 131) thereof.

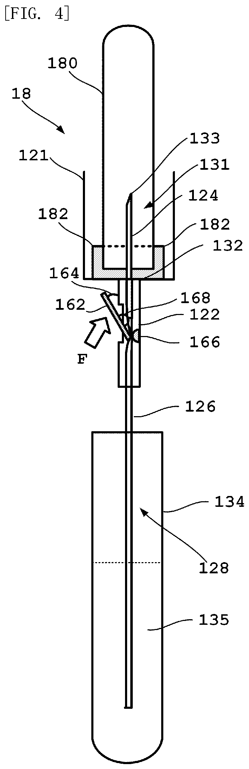

Fig. 4 is a diagram exemplifying a method for using the specimen collection kit 1 illustrated in Figs. 3A and 3B. According to Fig. 4, a specimen 135 such as human blood or urine is put in a test tube 134 in advance. In the state illustrated in Figs. 3A and 3B, a first end portion of the conduit 128 (that is, a distal end of the pipe 126) of the specimen collection device 12 is inserted into the specimen 135 from an opening of the test tube 134.

Next, when a user applies an appropriate force F to one end of the lever member 162, the other end of the lever member 162 rotates about the support members 168 in a direction opposite to the protruding member 166, closure of the pipe 126 is released so that the hollow portion of the conduit 128 is opened. Then, as indicated by a dotted arrow in Fig. 3C, the specimen 135 can flow through the inside of the conduit 128, that is, the needle tube 124 and the pipe 126. Here, the inside of the container main body 180 of the collection container 18 is held in a state of being depressurized with respect to the outside air in advance. Therefore, when the regulation by the regulating member 16 is released, the specimen 135 can flow into the pipe 126 from the first end portion of the conduit 128 until the air pressure inside the container main body 180 becomes equal to the external air pressure. Furthermore, the specimen 135 flows into the needle tube 124 connected to the pipe 126. Furthermore, the specimen 135 flows through the inside of the needle tube 124, and the specimen flows out to the inside of the container main body 180 from a distal end (second end portion) located inside the container main body 180. When the user stops the application of the force F to the lever member 162 at the time point when a necessary amount of the specimen 135 is collected inside the container main body 180, the pipe 126 is closed, and the collection of the specimen 135 ends.

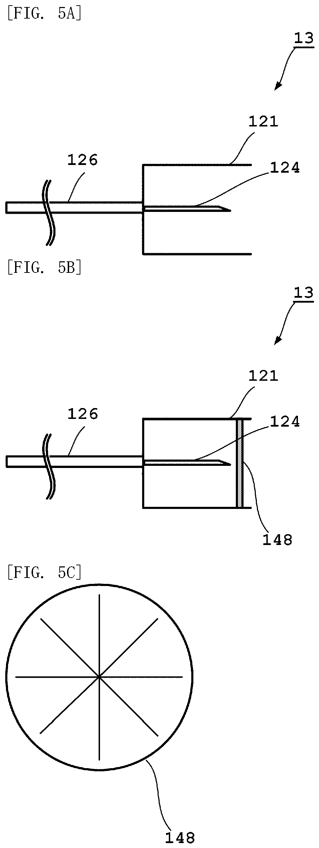

Here, Fig. 5A is a diagram illustrating a configuration of a specimen collection device 13 that can substitute for the specimen collection device 12 in the specimen collection kit 1. As illustrated in Fig. 5A, the specimen collection device 13 has a configuration in which the pipe holding member 122 is removed from the specimen collection device 12, and the second end portion of the pipe 126 is directly connected to a first end portion of the needle tube 124 via the main body 121 such that liquid or air does not leak. Such a connection of the pipe 126 to the main body 121 and the needle tube 124 can be implemented, for example, by bonding the needle tube 124 and the pipe 126 to the main body 121 and a second end portion of the needle tube 124 with an adhesive. The specimen collection device 13 has a simpler structure than the specimen collection device 12, and therefore can be manufactured at lower cost than the specimen collection device 12.

Fig. 5B is a diagram illustrating the configuration of the specimen collection device 13 that can substitute for the specimen collection device 12 in the specimen collection kit 1. As illustrated in Fig. 5B, the main body 121 of the collection container 18 of the specimen collection device 13 includes an abutting member 148 near an opening thereof. The abutting member 148 is formed into a shape along an outer periphery of the main body 121 and is disposed inside the main body 121. Here, Fig. 6C is a diagram illustrating the abutting member 148 that can be additionally disposed in each specimen collection device. As illustrated in Fig. 6C, the abutting member 148 is formed of an elastic material, has a plurality of cuts from a periphery toward a center, and has a shape in which a plurality of elastic pieces is included. Therefore, when the collection container 18 is inserted from an opening of the main body 121 and the collection container 18 abuts on the abutting member 148, the elastic pieces of the abutting member 148 are pressed and bent in a bottom surface direction of the main body 121 by elasticity. Then, when the collection container 18 is further inserted, the collection container 18 passes through an opening formed by the pressed and bent elastic pieces. Then, a distal end of the needle tube 124 is inserted into the lid 182 of the collection container 18. As described above, by using the abutting member 148, it is possible to prevent the needle tube 124 from being directly exposed to the outside before the collection container 18 is inserted, and it is possible to further improve safety.

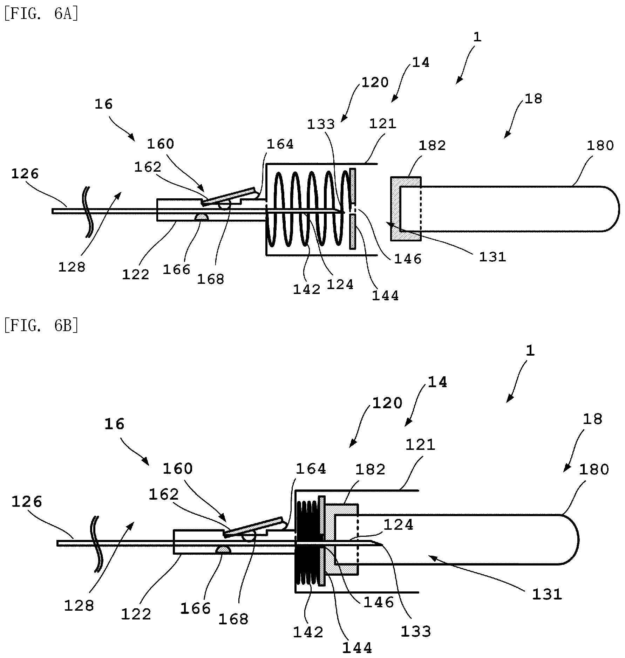

Figs. 6A and 6B are diagrams illustrating a configuration of a specimen collection device 14 that can substitute for the specimen collection device 12 in the specimen collection kit 1. As illustrated in Fig. 6A, the specimen collection device 14 includes the conduit 128, the holding member 120, and the regulating member 16 similarly to the specimen collection device 12. At a bottom of the main body 121 of the holding member 120, an elastic member 142 is disposed that biases the collection container 18 in a direction opposite to an insertion direction along a longitudinal direction when the collection container 18 is inserted into the main body 121 from an opening of the main body 121. Note that the elastic member 142 may be any of a leaf spring, a coil spring, a spiral spring, and a rubber as long as it can bias the collection container 18 in the direction via the elastic member 142, but Fig. 6A exemplifies a case where the elastic member 142 is a coil spring.

An abutting member 144 is disposed on an opening side of the elastic member 142 so as to be connected to the elastic member 142. When the collection container 18 is inserted into the main body 121, the abutting member 144 abuts on the lid 182 of the collection container 18. The abutting member 144 is formed into a substantially circular shape so as to conform to the shape of a cross section of the substantial cylinder of the main body 121, and an opening 146 is formed at a position of the abutting member 144 facing the needle tube 124 such that the needle tube 124 passes through the opening 146. When the collection container 18 is not inserted into the main body 121, the abutting member 144 is supported by the elastic member 142 so as to be located farther from a bottom of the main body 121 than the other end 133 of the needle tube 124 is. Note that, as an example, the abutting member 144 is formed of the same material as the holding member 120 and the lid 182.

In the state illustrated in Fig. 6A, when the collection container 18 is inserted in a direction of the opening of the main body 121, the collection container 18 first abuts on the abutting member 144. Thereafter, as illustrated in Fig. 6B, a force is applied to the collection container 18 in a direction toward the bottom of the main body 121 along a longitudinal direction, and the collection container 18 is inserted into the main body 121 from the opening of the main body 121. Then, the length of the elastic member 142 in the longitudinal direction becomes shorter, and the abutting member 144 moves in a direction of the bottom of the main body 121. As a result, the opening 146 of the abutting member 144 causes the needle tube 124 to pass in a direction of the lid 182 of the collection container 18. As a result, the needle tube 124 is inserted into the lid 182, and in this state, the specimen 135 that has flowed through the conduit 128 can flow into the container main body 180. As described above, if the elastic member 142 and the abutting member 144 are disposed inside the main body 121, the construction can be such that the needle tube 124 protrudes from the opening 146 to the opening side inside the main body 121 only when the specimen 135 is to be collected. That is, when the specimen 135 is not to be collected, the abutting member 144 covers the other end 133 of the needle tube 124, and therefore a needle stick accident by the needle tube 124 can be prevented.

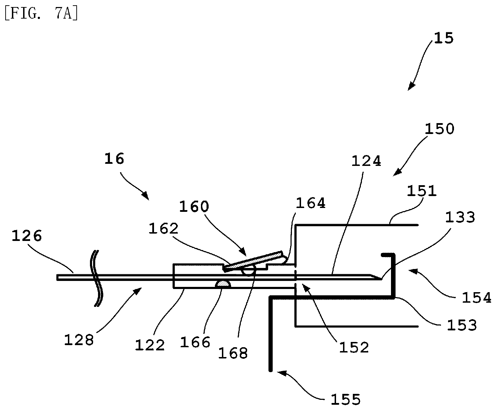

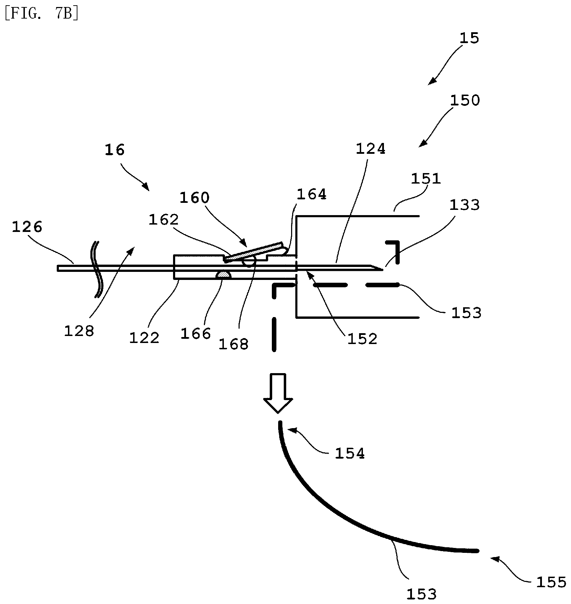

Figs. 7A and 7B are diagrams illustrating a configuration of a specimen collection device 15 that can substitute for the specimen collection device 12 in the specimen collection kit 1 according to the first embodiment. As illustrated in Fig. 7A, the specimen collection device 15 includes a holding member 150, and further includes the conduit 128 and the regulating member 16 similarly to the specimen collection devices 12 and 14. The holding member 150 includes a main body 151, a flexible and elongated needle cover 153 covering the other end 133 of the needle tube 124 at one end 154 when the specimen 135 is not to be collected, and the pipe holding member 122. The main body 151 is formed of a material similar to the main body 121, and has substantially the same shape as the main body 121. Note that, in a bottom of the main body 151, near a portion where the needle cover 153 and the pipe holding member 122 are connected to each other, an opening 152 is formed in order to pull out the other end portion 155 of the needle cover 153 to the outside of the main body 151.

Note that the needle cover 153 is formed of a material having flexibility and certain rigidity, such as a butyl rubber, a thermoplastic elastomer, polypropylene, polyethylene, polydimethylsiloxane (PDMS), or a combination thereof. In addition, the needle cover 153 is formed into a shape covering the entire needle tube 124 and particularly covering the other end 133 of the needle tube 124 with a material having a thickness of 1 to 5 mm. Furthermore, by forming the material covering the other end 133 of the needle tube 124 to be long, a possibility of occurrence of a needle stick accident can be reduced, and the specimen collection kit 1 using the needle cover 153 can be made safer.

As illustrated in Fig. 7B, before the specimen 135 is collected, a force is applied to the other end portion 155 of the needle cover 153, pulled out from the opening 152 to the outside of the main body 151, in a direction in which the needle cover 153 is pulled out from the main body 151. In such a case, the one end portion 154 of the needle cover 153 moves away from the other end 133 of the needle tube 124, and the entire needle cover 153 is pulled out from the opening 152 to the outside of the main body 151. When the needle cover 153 is pulled out and the collection container 18 is inserted into the main body 151, the other end 133 of the needle tube 124 is inserted into the lid 182 of the collection container 18. When the other end 133 is inserted into the lid 182, it becomes possible that the specimen 135 that has flowed through the conduit 128 flow into the container main body 180. Also in the specimen collection device 15, similarly to the specimen collection device 14, when the specimen 135 is to be not collected, the needle cover 153 covers the other end 133 of the needle tube 124, and therefore a needle stick accident caused by the needle tube 124 is prevented.

As described above, in the present embodiment, it is possible to provide a specimen collection device and a specimen collection kit capable of regulating outflow of a specimen into a container for collecting the specimen. In particular, the amount of a specimen to be collected in the collection container 18 can be easily adjusted by effectively regulating passage of the specimen through the conduit 128 by the regulating member 16. In addition, by using the needle cover 153, the abutting member, and the like, safety can be further enhanced.

### Second Embodiment

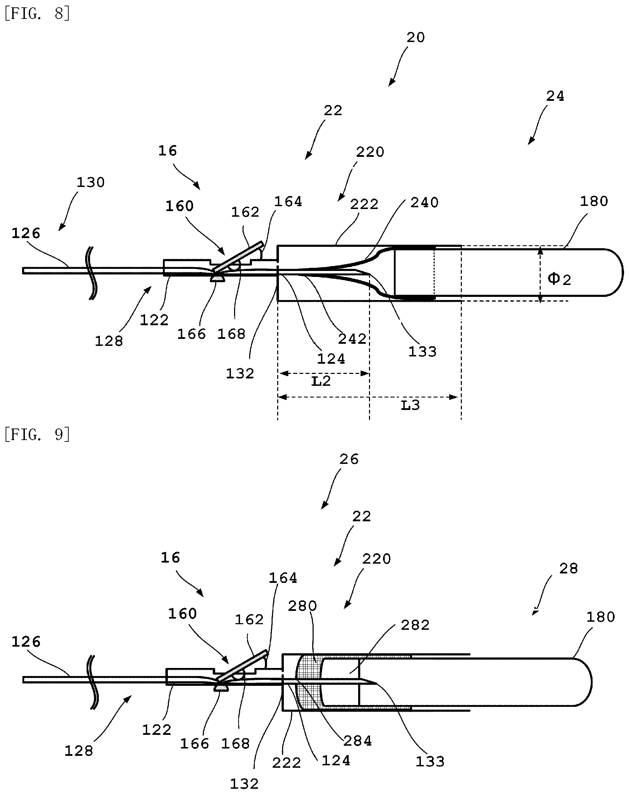

Next, a second embodiment will be described. Note that the second embodiment has a similar configuration to the first embodiment except for points specifically described below, and thus detailed description thereof will be omitted. Fig. 8 is a diagram exemplifying a configuration of a specimen collection device 22 according to the second embodiment and a specimen collection kit 20 including the specimen collection device 22. The specimen collection kit 20 includes the specimen collection device 22 and a collection container 24. The specimen collection device 22 includes a conduit 128, a holding member 220, and a regulating member 16. The holding member 220 includes a main body 222 and a pipe holding member 122. The collection container 24 includes a container main body 180 and a sealing member 240. Note that Fig. 8 illustrates a cross section of the specimen collection kit 20 in a state where the collection container 24 is inserted into the specimen collection device 22.

The main body 222 has a cylindrical shape similar to that of the main body 121, but is formed to be longer and thinner than the main body 121. That is, the main body 222 is formed into a substantially cylindrical shape similarly to the main body 121, but a length L3 of a cup-shaped portion thereof in a longitudinal direction is longer than the length L₁ (Fig. 2A) of the cup-shaped portion of the main body 121 in a longitudinal direction. Therefore, the lengths L1 and L3 have a relationship of L3 > L1. An inner diameter Φ2 of an opening of the main body 222 is narrower than the inner diameter Φ1 of the opening of the main body 121. Therefore, the inner diameters Φ1 and Φ2 have a relationship of Φ1 > Φ2. On the other hand, in the main body 222, the length L3 from a bottom thereof to the other end 133 of the needle tube 124 is the same as that in the main body 121. Therefore, a distance from the opening to the bottom of the main body 222 is longer than a distance from the opening of the main body 121 to the other end 133 of the needle tube 124. An inner diameter Φ2 of the opening of the main body 222 is shorter than the inner diameter Φ1 of the main body 121. Therefore, even when the specimen collection kit 20 is used by a medical worker with a thin finger, a possibility that the finger touches the other end 133 of the needle tube 124 is lower than that in a case where the specimen collection kit 1 is used. Therefore, when the holding member 220 of the specimen collection kit 20 is used, a possibility of occurrence of a needle stick accident is reduced.

In the collection container 24, unlike in the collection container 18, the container main body 180 is sealed by the sealing member 240 instead of the lid 182. The sealing member 240 is formed of a synthetic resin material such as a thermoplastic resin such as a polyolefin including polyethylene (PE), polypropylene (PP), polybutene, an ethylene-propylene copolymer, an ethylene-vinyl acetate copolymer, and a mixture thereof, a polyvinyl chloride resin, polyamide, a polyamide elastomer, polyester, a polyester elastomer, polyurethane (PU), or a fluororesin, or a material formed of a natural rubber (NR), a styrene butadiene rubber (SBR), a chloroprene rubber (CR), an acrylonitrile rubber (NBR), a butyl rubber (IIR), an ethylene propylene rubber (EPDM), a urethane rubber (U), a silicone rubber (SI), polydimethylsiloxane (PDMS), or a combination thereof. The sealing member 240 is formed into such a hollow shape that an outlet of an elongated funnel is closed, and an opening of the sealing member 240 is formed into a shape conforming to an opening of the container main body 180. The opening of the sealing member 240 is fitted to the opening of the container main body 180 to close and seal the opening of the container main body 180. As a result, a depressurized state of the inside of the container main body 180 is maintained by the sealing member 240 fitted to the opening of the container main body 180. On the other hand, an end portion of the sealing member 240 located on a side opposite to the opening is a head portion 242 that first enters the main body 222 when the collection container 24 is inserted into the main body 222.

Before collection of the specimen 135, the collection container 24 is provided to a medical person separately from the specimen collection device 22 in a state of not being inserted into the specimen collection device 22. When the specimen 135 is to be collected, first, the head portion 242 of the sealing member 240 and the other end 133 of the needle tube 124 are aligned to each other. Thereafter, when a force is applied to the collection container 24 in a direction of the bottom of the main body 222 along a longitudinal direction in a state where the specimen collection device 22 is fixed, the other end 133 of the needle tube 124 is inserted into the head portion 242 of the sealing member 240. Furthermore, the collection container 24 is inserted into the main body 222 until the head portion 242 of the sealing member 240 comes close to a bottom of the holding member 220. As a result, the other end 133 of the needle tube 124 reaches a hollow portion inside the container main body 180 or the sealing member 240 from the head portion 242. In such a state, when a force F is applied to the lever member 162 as illustrated in Fig. 3C, closure of the pipe 126 of the conduit 128 is released, and the specimen 135 is caused to flow out into the container main body 180 of the collection container 24 through the conduit 128.

Hereinafter, a modification of the second embodiment will be described. Fig. 9 is a diagram exemplifying a configuration of a specimen collection kit 26 including the specimen collection device 22 as a modification of the second embodiment. As illustrated in Fig. 9, the specimen collection kit 26 includes the specimen collection device 22 and a collection container 28. The collection container 28 includes the container main body 180 and a sealing member 280. Note that Fig. 9 illustrates a cross section of the specimen collection kit 26 in a state where the sealing member 240 is attached to the specimen collection device 22.

The specimen collection kit 26 has a configuration in which the collection container 24 of the specimen collection kit 20 is replaced with the collection container 28. In the collection container 28, unlike in the collection container 24, the container main body 180 is sealed by the sealing member 280 formed of a material similar to the sealing member 240, instead of the sealing member 240. Note that, unlike the sealing member 240, the sealing member 280 has a shape like a cap of the container main body 180. That is, the sealing member 280 covers an opening of the container main body 180 from the outside, and has a gently protruding shape with respect to a bottom of the main body 222 when being inserted into the main body 222. Note that a part of the inside of the sealing member 280 forms a hollow portion 282 located closer than an opening of the sealing member 280. A portion of the sealing member 280 having a protruding shape with respect to the main body 222 becomes a head portion 284 of the sealing member 280 that first enters the main body 222 when the collection container 28 is inserted into the main body 222.

Before collection of the specimen 135, the collection container 28 is provided to a medical person separately from the specimen collection device 22 in a state of not being inserted into the specimen collection device 22, similarly to the collection container 24. When the specimen 135 is to be collected, first, the head portion 284 of the sealing member 280 and the opening of the main body 222 are aligned to each other. Thereafter, a force is applied to the collection container 28 in a direction of the bottom of the main body 222 along a longitudinal direction in a state where the specimen collection device 22 is fixed. Then, the other end 133 of the needle tube 124 is inserted into the head portion 284 of the sealing member 240. In this state, the collection container 28 is inserted into the main body 222 until the head portion 284 of the sealing member 280 comes close to a bottom of the holding member 220. As a result, the other end 133 of the needle tube 124 reaches a hollow portion inside the container main body 180 or the hollow portion 282 from the head portion 284. In such a state, when a force F is applied to the lever member 162 as illustrated in Fig. 3C, closure of the pipe 126 of the conduit 128 is released, and the specimen 135 is caused to flow out into the container main body 180 of the collection container 28 through the conduit 128.

### Third Embodiment

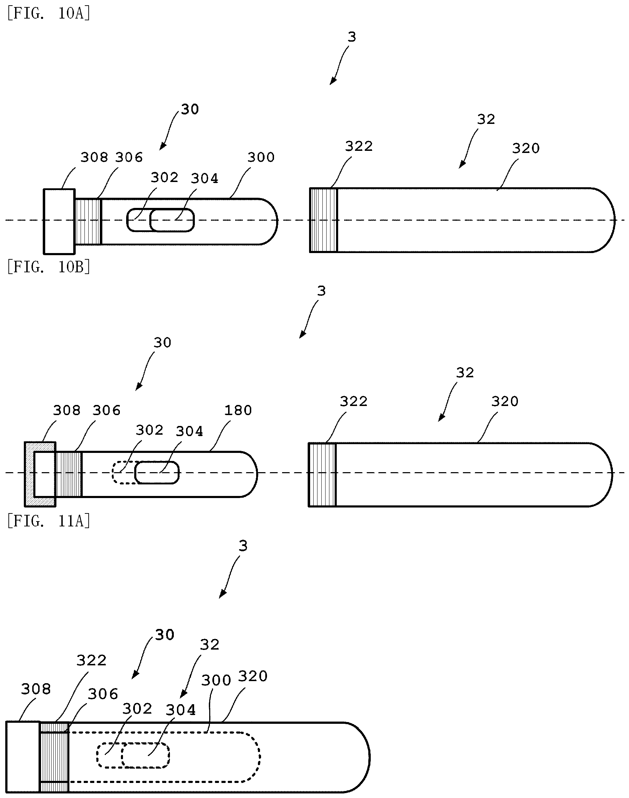

Next, a third embodiment will be described in detail. Note that the third embodiment has a similar configuration to the first and second embodiments except for points specifically described below, and thus detailed description thereof will be omitted. Figs. 10A and 10B are diagrams exemplifying a configuration of a specimen cylinder 3 according to the third embodiment that can be used for specimen collection by the specimen collection devices 12, 14, 15, and 22 illustrated in the first and second embodiments. Specifically, Fig. 10A illustrates a side view of a specimen collection tube 30 and a specimen collection component 32 included in the specimen cylinder 3, and Fig. 10B illustrates a cross-sectional view of the specimen collection tube 30 and the specimen collection component 32 included in the specimen cylinder 3.

According to Figs. 10A and 10B, the specimen cylinder 3 includes a specimen collection tube 30 in which a specimen is first collected, and a specimen collection component 32 connected to the specimen collection tube 30 so as to cover the specimen collection tube 30. The specimen collection tube 30 includes a collection tube main body 300, two openings 302 and 304 having different sizes, a screw structure 306, and a lid 308. The collection tube main body 300 has a longitudinally long cylindrical shape with an open top like a test tube. Openings 302 and 304 are formed on a side surface connecting a bottom and the upper opening of the collection tube main body 300. A specimen is mainly collected from an opening having a larger opening area out of the openings 302 and 304, and the specimen is discarded from a smaller opening. For example, when the specimen is urine, urine is released toward the larger opening, and the specimen flows into the collection tube main body 300 from the opening. The screw structure 306 is formed adjacent to the lid 308 on the side surface of the collection tube main body 300 near an opening of the collection tube main body 300. The lid 308 covers and closes the opening of the collection tube main body 300.

The specimen collection component 32 includes a cylinder main body 320 having a longitudinally long cylindrical shape with an open top, such as a test tube having a larger inner diameter than an outer diameter of the collection tube main body 300 of the specimen collection tube 30. A screw structure 322 including a groove conforming to the screw structure 306 is formed on an inner wall of the cylinder main body 320 near an opening of the cylinder main body 320. The collection tube main body 300 and the cylinder main body 320 are formed of, for example, a material similar to the container main body 180 of the specimen collection kit 1 or a material similar to the main body 121 and the pipe holding member 122 of the specimen collection device 12.

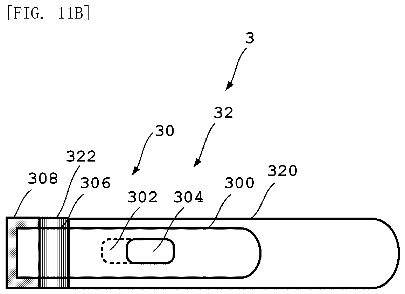

Figs. 11A and 11B are diagrams exemplifying a configuration of the specimen cylinder 3 according to the third embodiment used for specimen collection by the specimen collection kit 1 illustrated in Figs.3A to 3C. Specifically, Fig. 11A illustrates a side view of the specimen collection tube 30 and the specimen collection component 32 included in the specimen cylinder 3, and Fig. 11B illustrates a cross-sectional view of the specimen collection tube 30 and the specimen collection component 32 included in the specimen cylinder 3.

Here, although not particularly illustrated in Figs. 11A and 11B, a screw structure similar to the screw structure 306 may also be formed on an upper portion of the lid 308 (that is, a side opposite to the side on which the screw structure 306 is formed). By forming such a screw structure, the screw structure 322 of the specimen collection component 32 is fitted to the screw structure formed on the upper portion of the lid 308, and the specimen collection component 32 can be connected to an upper portion of the specimen collection tube 30. As a result, for example, at the time of urine collection, a user holds the specimen collection component 32 and discharges urine toward any one of the openings 302 and 304 of the specimen collection tube 30, whereby it is possible to reduce a risk of contaminating the user's hand or the like with urine.

As described above, after a specimen is collected in the specimen collection tube 30, as illustrated in Figs. 11A and 11B, the collection tube main body 300 of the specimen collection tube 30 is inserted from the opening of the cylinder main body 320 of the specimen collection component 32. Furthermore, the collection tube main body 300 of the specimen collection tube 30 and the cylinder main body 320 of the specimen collection component 32 are screwed and fixed by the screw structure 306 of the specimen collection tube 30 and the screw structure 322 of the specimen collection component 32. As described above, by covering and fixing the specimen collection tube 30 with the specimen collection component 32, it is possible to prevent a specimen collected in the collection tube main body 300 of the specimen collection tube 30 from leaking to the outside.

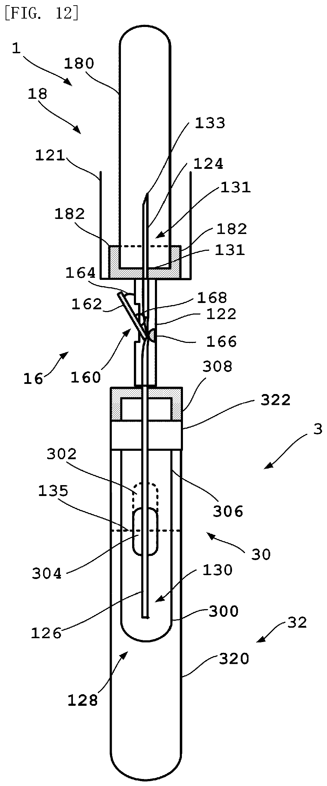

Fig. 12 is a diagram illustrating a method for collecting a specimen, put in the specimen cylinder 3 illustrated in Figs. 11A and 11B, using the specimen collection kit 1. According to Fig. 12, in a state where the specimen 135 is collected inside the collection tube main body 300 of the specimen collection tube 30, the specimen collection component 32 is fixed so as to cover the specimen collection tube 30 via the lid 308.

In this state, in a state where the conduit 128 is closed, a first end portion of the conduit 128 of the specimen collection kit 1 is inserted into the specimen collection tube 30 via the lid 308. At this time, the first end portion of the conduit 128 of the specimen collection kit 1 is inserted until reaching the specimen 135. As a result, the specimen collection kit 1 and the specimen cylinder 3 are connected to each other. When a user applies an appropriate force F to one end of the lever member 162 of the regulating member 16 in a state where the specimen collection kit 1 and the specimen cylinder 3 are connected to each other, the other end of the lever member 162 rotates about the support members 168 in a direction opposite to the protruding member 166. As a result, the closure of the conduit 128 by the regulating member 16 is released, and the hollow portion of the conduit 128 is opened. Here, the inside of the container main body 180 of the collection container 18 is held in a state of being depressurized with respect to the outside air in advance. Therefore, when the regulation by the regulating member 16 is released, it becomes possible for the specimen 135 to flow into the container main body 180 from a distal end (second end portion) located inside the container main body 180 until the air pressure inside the container main body 180 becomes the same as the external air pressure. When a necessary amount of the specimen 135 is collected inside the container main body 180 and the user stops the application of the force F to the lever member 162, the conduit 128 is closed again, and the collection of the specimen 135 into the specimen collection kit 1 ends.

As described above, similarly to the first and second embodiments, in the present embodiment, it is possible to provide a specimen collection device and a specimen collection kit capable of regulating outflow of a specimen into a container for collecting the specimen. In particular, the amount of a specimen to be collected in the collection container 18 can be easily adjusted by effectively regulating passage of the specimen through the conduit 128 by the regulating member 16. In addition, according to the specimen collection kit 1 of the present embodiment, it is possible to collect specimens from various containers and the like.

### Fourth Embodiment

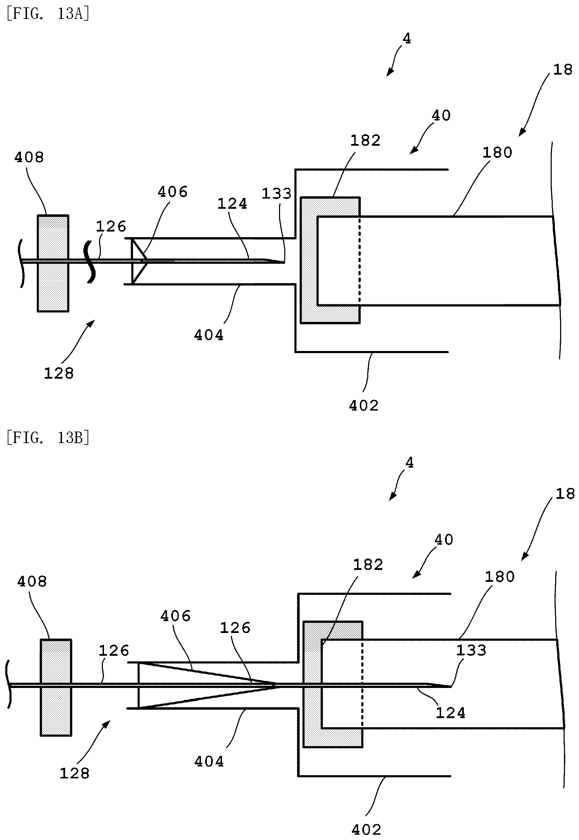

Next, a fourth embodiment will be described. Note that the fourth embodiment has a similar configuration to the first to third embodiments except for points specifically described below, and thus detailed description thereof will be omitted. Figs. 13A to 13C are diagrams exemplifying a cross section of a specimen collection device 40 according to a fourth embodiment and a part of a specimen collection kit 4 including the specimen collection device 40 in an enlarged manner. As illustrated in Fig. 13A, the specimen collection kit 4 includes the specimen collection device 40 and a collection container 18. The specimen collection device 40 includes a conduit 128 and a holding member 400. The holding member 400 includes a main body 402, a pipe holding member 404, a backflow preventing member 406, and a pressing member 408.

The main body 402 is formed of a material similar to that of the main body 121, and is formed into a cylindrical shape similarly to the main body 121 of the specimen collection kit 1. The main body 402 is formed of a material similar to the pipe holding member 122 of the specimen collection kit 1. A bottom of the main body 402 has an opening into which the collection container 18 is inserted from a lid 182 side, and the inserted lid 182 abuts on the bottom. The pipe holding member 404 is formed of a material similar to that of the pipe holding member 122, and is connected to the opening of the bottom of the main body 402. The pipe holding member 404 is formed into a substantially cylindrical shape similarly to the pipe holding member 122, and has an inner diameter larger than an inner diameter of the pipe holding member 122. The conduit 128 passes through the pipe holding member 404, and the conduit 128 is connected to an inner wall of the pipe holding member 404 via the backflow preventing member 406 formed in a film shape having high elasticity.

The backflow preventing member 406 is formed of a synthetic resin material such as a thermoplastic resin such as polypropylene (PP), polystyrene (PS), an acrylonitrile-styrene resin (AS), an acrylonitrile-butadiene-styrene resin (ABS), polyvinyl chloride (PVC), a methacrylic resin (PMMA), or polyethylene terephthalate (PET), or a material formed of a natural rubber (NR), a styrene butadiene rubber (SBR), a chloroprene rubber (CR), an acrylonitrile rubber (NBR), a butyl rubber (IIR), an ethylene propylene rubber (EPDM), a urethane rubber (U), a silicone rubber (SI), polydimethylsiloxane (PDMS), waterproof paper, a vegetable plastic, or a combination thereof.

The backflow preventing member 406 closes a space between the conduit 128 and an inner wall of the pipe holding member 404 without a gap. Note that the backflow preventing member 406 does not need to be formed into a film shape, and only needs to be formed into a shape that can close a space between the needle tube 124 and the inner wall of the pipe holding member 404 without a gap. The pressing member 408 is attached around a pipe 126 of the conduit 128, the pressing member being used in order to apply a force to press the conduit 128 in a direction of a bottom surface of the main body 402 along a longitudinal direction. Note that, as illustrated in Fig. 13A, unless a pressing force is applied to the pressing member 408, the needle tube 124 of the conduit 128 is housed in the pipe holding member 404 over the entire length by elasticity of the backflow preventing member 406.

Before collection of a specimen 135, the collection container 18 is provided to a medical person separately from the specimen collection device 40 in a state of not being inserted into the specimen collection device 40, similarly to the collection container 18 of the specimen collection kit 1 and the like. When the specimen 135 is to be collected, first, a central portion of the lid 182 of the collection container 18 and an opening connected to a hollow portion of the pipe holding member 404 at a bottom of the main body 402 are aligned to each other. Thereafter, a force in a direction of the collection container 18 is applied to the pressing member 408 along a longitudinal direction while the position of the collection container 18 is maintained. Then, as illustrated in Fig. 13B, the conduit 128 moves in the direction of the collection container 18. With the movement of the collection container 18, the backflow preventing member 406 largely extends in a direction of the bottom of the main body 402 while closing the space between the conduit 128 and the inner wall of the pipe holding member 404, and the other end 133 of the needle tube 124 of the conduit 128 is inserted into the lid 182. As a result, the other end 133 of the needle tube 124 passes through the lid 182 and reaches the hollow portion inside the container main body 180, and the specimen 135 is caused to flow out into the container main body 180 of the collection container 28 through the conduit 128.

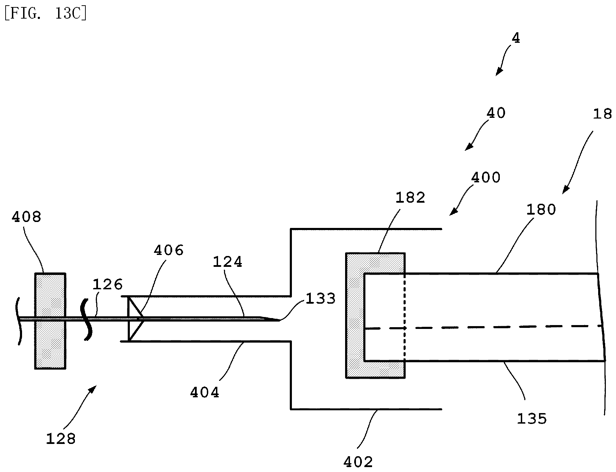

When a force is applied to the pressing member 408 in a direction away from the collection container 18 along a longitudinal direction, the other end 133 of the needle tube 124 comes out of the lid 182 as illustrated in Fig. 13C. Furthermore, when the collection container 18 is moved in a direction away from a bottom surface of the main body 402, the collection container 18 containing the specimen 135 is taken out from the specimen collection device 40.

### Fifth Embodiment

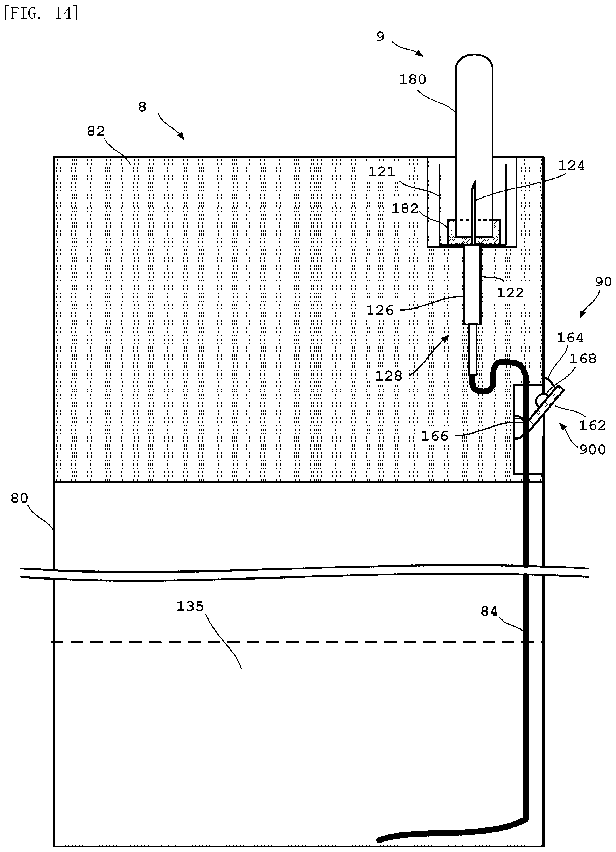

Next, a fifth embodiment will be described in detail. Note that the fifth embodiment has a similar configuration to the first to fourth embodiments except for points specifically described below, and thus detailed description thereof will be omitted. Fig. 14 is a diagram exemplifying a cross section of a specimen cup 8 in which a specimen collection kit 1 according to the fifth embodiment is used. As illustrated in Fig. 14, the specimen cup 8 includes a specimen cup main body 80 and a lid 82. A specimen collection kit 9 and a regulating member 90 are attached to a recess formed in the lid 82. The specimen collection kit 9 has a configuration in which an elongated tube 84 is connected to a first end portion of a conduit 128 such that air or a specimen does not leak. The tube 84 is connected to the first end of the conduit 128 in this manner by, for example, widening a second end portion of the tube 84 and covering the outside of a first end portion 130 of the conduit 128. Alternatively, this connection may be made by disposing a member for attaching the tube 84 at the first end portion of the conduit 128. The specimen cup 8 is suitable, for example, for collecting a relatively large amount of specimen.

The tube 84 is typically formed of a flexible material, and as an example thereof, the tube 84 is formed of a synthetic resin material such as a thermoplastic resin such as polyethylene (PE), polypropylene (PP), polystyrene (PS), an acrylonitrile-styrene resin (AS), an acrylonitrile-butadiene-styrene resin (ABS), polyvinyl chloride (PVC), a methacrylic resin (PMMA), or polyethylene terephthalate (PET), or a material formed of waterproof paper, a vegetable plastic, or a combination thereof.

A recessed portion 900 is formed in the lid 82 in a size that can house a regulating member 16 of the specimen collection kit 1, and the regulating member 90 is housed in the recessed portion 900. The regulating member 90 includes a lever member 162, an elastic member 164, a protruding member 166, and a support members 168 disposed in the recessed portion 900 and a periphery thereof. The tube 84 is drawn into the recessed portion 900, and the tube 84 passes between the lever member 162 and the protruding member 166. Therefore, also in the regulating member 90, as in the regulating member 16, flow of a specimen in the conduit 128 and the tube 84 can be regulated by application of a force F to the lever member 162 by a user.

One end of the tube 84 (an end located in a direction opposite to an end connected to the first end portion 130 of the conduit 128) is guided into the specimen 135 collected in the specimen cup main body 80. In this state, when a force F is applied to the lever member 162 of the regulating member 90, the specimen 135 flows through the tube 84 and the conduit 128, flows out into the container main body 180, and is accumulated inside the container main body 180. When a sufficient amount of the specimen 135 is accumulated inside the container main body 180 and the application of the force F to the lever member 162 by the user ends, the flow of the specimen 135 in the tube 84 and the conduit 128 is stopped, and the collection of the specimen 135 ends.

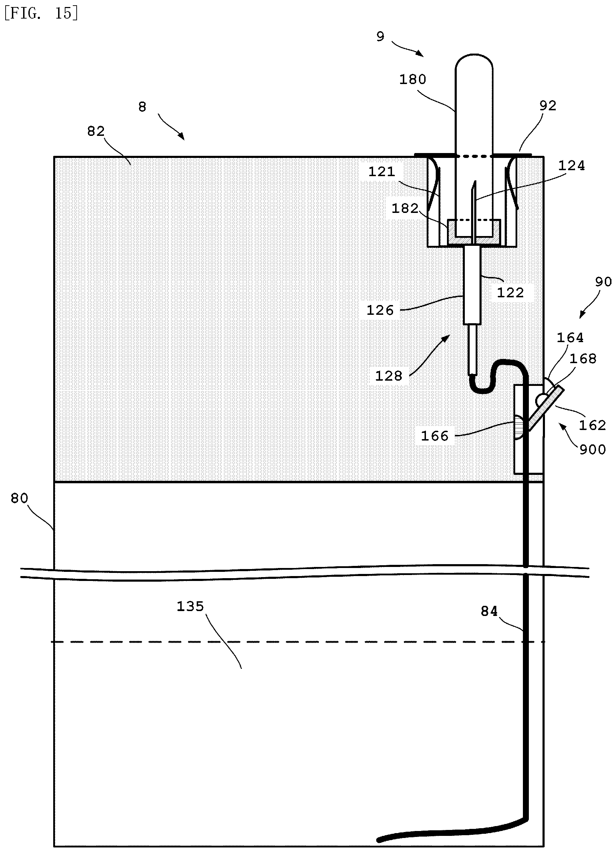

Hereinafter, a modification of the fifth embodiment will be described. Fig. 15 is a diagram exemplifying a modification of the fifth embodiment. As illustrated in Fig. 15, in this modification, in the lid 82 of the specimen cup 8, a lid 92 is attached to an opening of a recess to which the main body 121 of the specimen collection kit 9 and the like are attached. When the collection container 18 is not inserted into the main body 121, the opening is covered with the lid 92. Note that the lid 92 is formed of an elastic material similarly to the abutting member 148 illustrated in Fig. 6C, and has a plurality of cuts from a periphery toward a center. When the collection container 18 is inserted into the main body 121 from the lid 92, a cut portion of the lid 92 is warped, and the collection container 18 passes therethrough. By attaching such a lid 92 to the opening of the recess to which the main body 121 of the specimen collection kit 9 and the like are attached, a needle stick accident caused by the needle tube 124 is prevented.

### Modification

In each of the above embodiments, the case where a structure for elastically closing at least a part of the conduit 128 by the lever member 162 is included as the structure of the regulating member 16 has been described. The elastic member 184 disposed in the regulating member 16 only needs to be able to bias the lever member 162 in a desired direction, is not limited to a leaf spring, and may be any of a coil spring, a spiral spring, a rubber, and the like. In addition, the regulating member 16 only needs to be able to regulate flow of a specimen, and can adopt various structures such as a clamp, a closed connector, a clip, a stopcock, and a valve instead of or in addition to the lever member 162 as described above.

As described above, similarly to the first to fourth embodiments, according to the present embodiment, it is possible to provide a specimen collection device and a specimen collection kit capable of regulating outflow of a specimen into a container for collecting the specimen. In particular, the amount of a specimen to be collected in the collection container 18 can be easily adjusted by effectively regulating passage of the specimen through the conduit 128 by the regulating member 16. In addition, according to the specimen collection kit 1 of the present embodiment, it is possible to collect specimens from various containers and the like.

Although several embodiments have been described above, these embodiments have been presented as examples, and are not intended to limit the scope of the invention. These novel embodiments can be implemented in various other forms, and various omissions, substitutions, and changes can be made without departing from the gist of the invention. These embodiments and modifications thereof are included in the scope and gist of the embodiments and are included in the invention described in the claims and a scope equivalent thereto.

### Reference Signs List

1, 20, 26, 4, 9 Specimen collection kit
12, 13, 14, 15, 22, 28, 40 Specimen collection device
120, 150, 220 Holding member
121, 151, 222, 402 Main body
122, 404 Pipe holding member
124 Needle tube
126 Pipe
128 Conduit
130, 131 End portion
133, 134 End
134 Test tube
135 Specimen
146, 160 Opening
142 Elastic member
144, 148 Abutting member
153 Needle cover
16, 90 Regulating member
162 Lever member
164 Elastic member
166 Protruding member
168 Support member
18, 24, 28 Collection container
180 Container main body
182, 82, 92 Lid
240, 280 Sealing member
3 Specimen cylinder
30 Specimen collection tube
300 Collection tube main body
302, 304 Opening
306, 322 Screw structure
32 Specimen collection container
320 Cylinder main body
322 Screw structure
406 Backflow preventing member
408 Pressing member
8 Specimen cup
80 Specimen cup main body
84 Tube
900 Recessed portion

## Claims

1. A specimen collection device comprising:
a conduit configured to cause a specimen flowing into a first end portion from an outside of a container having an inside depressurized for collecting the specimen to flow out into the container from a second end portion disposed so as to be opposite to the first end portion;
a holding member configured to hold the container and the conduit; and
a regulating member configured to cause the specimen to flow out to the container when being opened and regulate outflow of the specimen to the container when being closed.

2. The specimen collection device according to claim 1, wherein the specimen is a body fluid derived from a living body.

3. The specimen collection device according to claim 1, wherein the conduit includes a hollow pipe and a hollow needle tube to be inserted into the container from a lid of the container.

4. The specimen collection device according to claim 3, wherein the regulating member regulates, in the pipe, outflow of the specimen into the container.

5. The specimen collection device according to claim 3, wherein the regulating member regulates outflow of the specimen into the container by closing the pipe.

6. The specimen collection device according to claim 3, wherein
the pipe is configured to be crushed at least in a short direction of a cross section of the pipe by pressurization, and
the regulating member regulates outflow of the specimen into the container by pressurizing the pipe in the short direction.

7. The specimen collection device according to claim 1, wherein the regulating member is disposed in the holding member.

8. The specimen collection device according to claim 1, wherein
the first end portion is connected to a tube having a distal end drawn into another container different from the container, and
the regulating member regulates outflow of the specimen into the container in the tube.

9. The specimen collection device according to claim 8, wherein the regulating member is disposed away from the holding member.

10. A specimen collection kit comprising:
a collection container having an inside depressurized for collecting a specimen and sealed with a lid; and
the specimen collection device according to claim 1.
